# EUROPEAN PATENT APPLICATION

(11) **EP 4 108 271 A1**
(43) Date of publication of application: **28.12.2022**
(21) Application number: 21763658.8
(22) Date of filing: 04.03.2021
(51) Int. Cl.: A61M 5/145, A61M 5/315

(54) **SYRINGE PUMP**

(30) Priority: 05.03.2020 JP 2020038011
(71) Applicant: Nipro Corporation, Osaka-shi, Osaka 531-8510 (JP)
(72) Inventor: HABATA, Motoharu, Chuo-shi, Yamanashi 409-3801 (JP)
(74) Representative: Prüfer & Partner mbB Patentanwälte · Rechtsanwälte
(86) International application number: PCT/JP2021/008331
(87) International publication number: WO 2021/177389

(57) **Abstract**

A syringe pump (100) is applied to a syringe (200) that includes a barrel (210) and a plunger rod (220) which is fitted inside the barrel (210). A flange (221) is provided at a rear end of the plunger rod (220). The syringe pump (100) includes a clamping mechanism (150, 160) which clamps and fixes the flange (221) of the plunger rod (220) in the thickness direction of the flange (221). The clamping mechanism (150, 160) includes a pushing portion (150) which includes a pressing face facing a rear end face of the flange (221), and a gripping portion (160) which is movable in the thickness direction and connected to the pushing portion (150) so as to form a gap with the pressing face in the thickness direction, and is configured to work in cooperation with the pushing portion (150) so as to fix the flange (221). The pushing portion (150) includes a locking mechanism (153A, 155A) which locks the movement of the gripping portion (160) in a direction away from the pressing face with the flange (221) being fixed.

## Description

### TECHNICAL FIELD

The present disclosure relates to a syringe pump.

### BACKGROUND ART

A syringe pump is used to administer an accurate amount of liquid medicine from a syringe. For example, PTL 1 (Japanese Patent Laying-Open No. 2017-51548) and PTL 2 (Japanese Patent Laying-Open No. 2019-10502) disclose a technique for suppressing a siphoning phenomenon which causes the liquid medicine in the syringe to be administered without being controlled by the syringe pump.

### CITATION LIST

### PATENT LITERATURE

PTL 1: Japanese Patent Laying-Open No. 2017-51548
PTL 2: Japanese Patent Laying-Open No. 2019-10502

### SUMMARY OF INVENTION

### TECHNICAL PROBLEM

There is known a mechanism that clamps a flange formed at a rear end of a plunger rod of a syringe in a thickness direction of the flange. By providing a function of sliding a gripping portion in the thickness direction of the flange so as to expand a gap formed therebetween, it is possible to improve the efficiency of installing the syringe in the syringe pump.

However, when a force is generated to draw the plunger rod under a negative pressure condition, the gripping portion will move together with the plunger, which makes it impossible to prevent the plunger rod from moving unintendedly.

It is an object of the present disclosure to provide a syringe pump capable of preventing a plunger rod from moving unintendedly even under a negative pressure condition.

### SOLUTION TO PROBLEM

The syringe pump according to the present disclosure is applied to a syringe that includes a barrel and a plunger rod which is fitted inside the barrel. A flange is provided at a rear end of the plunger rod. The syringe pump includes a clamping mechanism which clamps and fixes the flange of the plunger rod in a thickness direction of the flange. The clamping mechanism includes a pushing portion which includes a pressing face facing a rear end face of the flange, and a gripping portion which is movable in the thickness direction and connected to the pushing portion so as to form a gap with the pressing face in the thickness direction, and is configured to work in cooperation with the pushing portion so as to fix the flange. The pushing portion includes a locking mechanism which locks the movement of the gripping portion in a direction away from the pressing face with the flange being fixed.

In one aspect, the syringe pump further includes an operation member for operating the gripping portion. The pushing portion further includes a transmission mechanism which transmits an operation force from the operation member to the gripping portion, and a casing which forms the pressing face and houses the transmission mechanism, and the locking mechanism is formed between the transmission mechanism and the casing.

In one aspect of the syringe pump, the locking mechanism includes a tapered face formed on the casing, and an abutment member provided on the transmission mechanism and configured to abut against the tapered face.

In one aspect of the syringe pump, the gripping portion includes a finger member rotatable about an axis in the thickness direction with respect to the pushing portion, the transmission mechanism includes a rotating member which rotates together with the finger member, and the abutment member is formed on the rotating member.

In one aspect of the syringe pump, the pushing portion further includes a bias member configured to bias the transmission mechanism so as to draw the gripping portion toward the pressing face.

### ADVANTAGEOUS EFFECTS OF INVENTION

According to the syringe pump of the present disclosure, it is possible to prevent the plunger rod from moving unintendedly even under a negative pressure condition.

### BRIEF DESCRIPTION OF DRAWINGS

Fig. 1 is a first perspective view illustrating a syringe pump according to an embodiment of the present disclosure;
Fig. 2 is a view illustrating the syringe pump of Fig. 1 when viewed from the Y-axis direction;
Fig. 3 is a second perspective view illustrating a syringe pump according to one embodiment of the present disclosure;
Fig. 4 is a perspective view illustrating an appearance of a syringe to be installed in the syringe pump illustrated in Figs. 1 to 3;
Fig. 5 is a perspective view illustrating an internal structure of a pushing portion of the syringe pump illustrated in Figs. 1 to 3;
Fig. 6 is a view illustrating a transmission mechanism provided in the pushing portion illustrated in Fig. 5 when viewed from the Z-axis direction; and
Fig. 7 is a cross-sectional view of the pushing portion illustrated in Fig. 5 cut along the Y-axis direction.

### DESCRIPTION OF EMBODIMENTS

An embodiment of the present invention will be described below. It should be noted that the same or equivalent portions in the drawings will be denoted by the same reference numerals, and the description thereof will not be repeated.

It should be noted that unless otherwise specified, the scope of the present disclosure is not limited to the number, the amount or the like mentioned in the embodiment to be described below. In the following embodiment, unless otherwise specified, each component is not necessarily essential to the present disclosure.

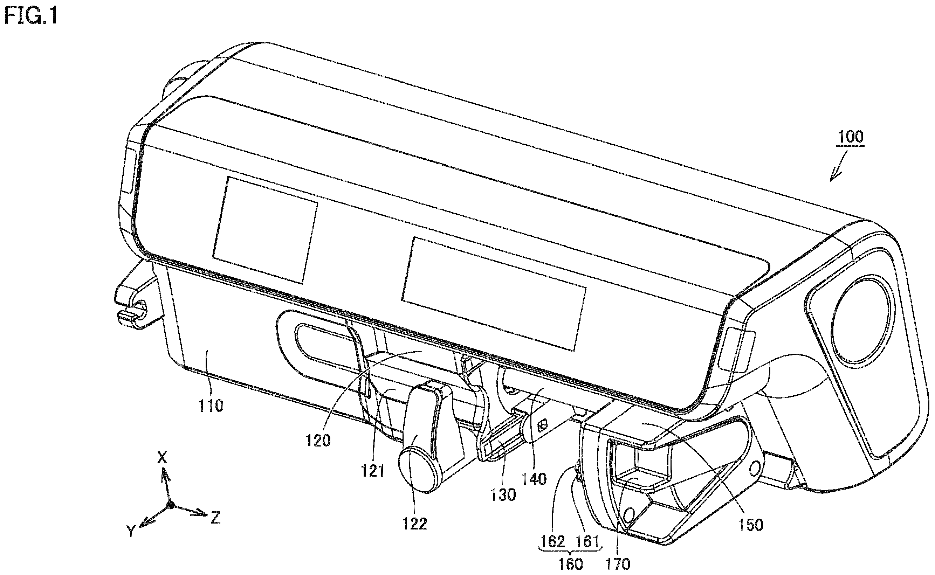

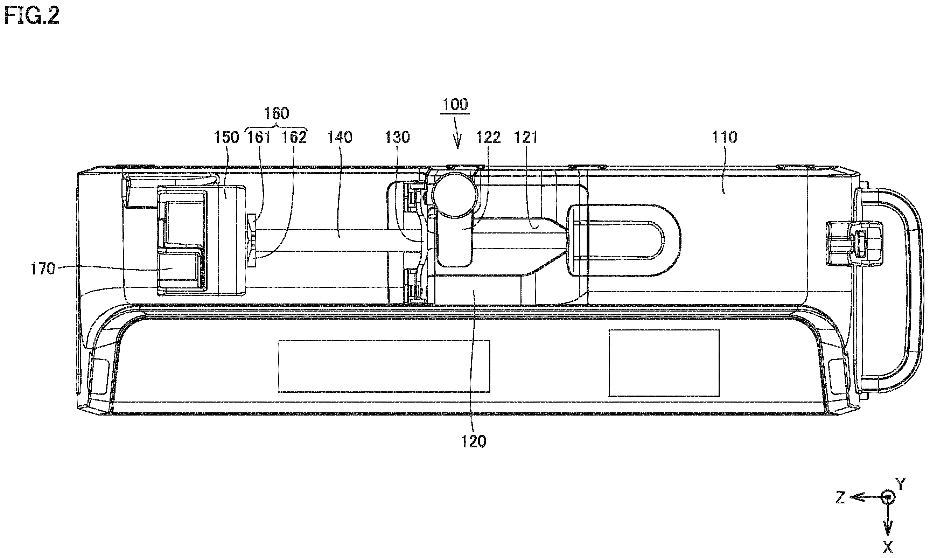

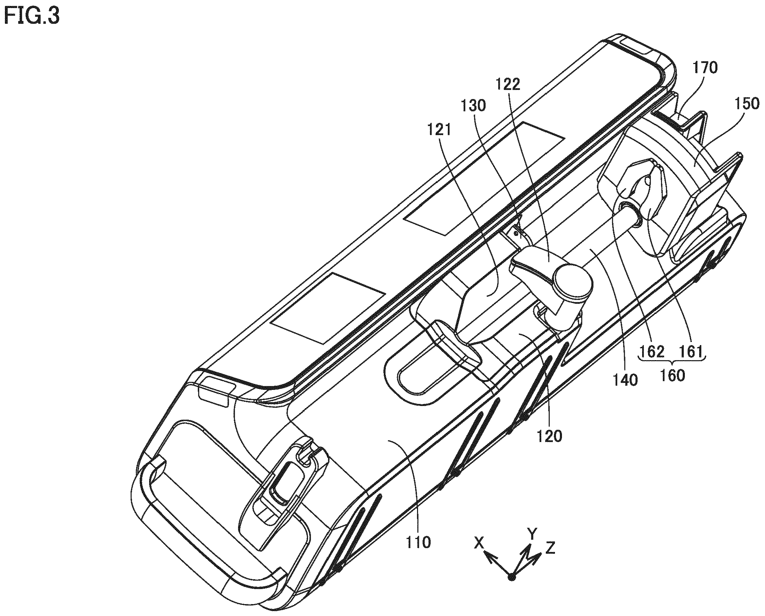

Figs. 1 to 3 each illustrate a syringe pump according to an embodiment of the present disclosure. The syringe pump 100 illustrated in each of Figs. 1 to 3 is viewed from different directions. The X-axis, the Y-axis and the Z-axis illustrated in Figs. 1 to 3 are orthogonal to each other.

As illustrated in Figs. 1 to 3, the syringe pump 100 includes a main body 110, a syringe mounting member 120, a barrel flange retainer 130, a slide shaft 140, a pushing portion 150, a gripping portion 160, and an operation member 170.

The main body 110 is provided with a drive unit and a control unit that controls the drive unit. The drive unit includes a motor that drives the slide shaft 140 and a reduction gear that is connected to the motor.

A syringe to be described later is mounted on the syringe mounting member 120. The syringe mounting member 120 is provided at one side of the main body 110. The syringe mounting member 120 includes a concave member 121 and a syringe clamp 122.

The concave member 121 has a concave face which curves into an arc shape in the cross section, and is configured to extend in the central axis direction of the arc. The syringe clamp 122 is provided to face the concave member 121 and is configured to be brought into contact or out of contact with the concave member 121. The syringe clamp 122 has a concave face which is provided at the side facing the concave member 121 and is configured to curve into an arc shape in the opposite direction to the concave face of the concave member 121. The concave face of the concave member 121 and the concave face of the syringe clamp 122 clamp the barrel therebetween, whereby the syringe is fixed in the syringe mounting member 120. The barrel flange retainer 130 is provided at an end of the syringe mounting member 120. After the syringe is installed in the syringe pump, the barrel flange retainer 130 abuts against a flange (barrel flange) provided at the end of the barrel.

One end of the slide shaft 140 is connected to the drive unit of the main body 110, and the other end thereof is connected to the pushing portion 150. The slide shaft 140 is slidable in the axial direction of the slide shaft 140. The axial direction of the slide shaft 140 is substantially parallel to the extending direction of the concave member 121. The configuration of the pushing portion 150 will be described later.

The pushing portion 150 and the gripping portion 160 constitute a "clamping mechanism" that clamps and fixes the flange of the plunger rod of the syringe in the thickness direction of the flange (the Z-axis direction).

The grip member 160 includes a pair of finger members 161 and 162. Each of the pair of finger members 161 and 162 is connected to the operation member 170 with the pushing portion 150 interposed therebetween.

When the operation member 170 is in a state as illustrated in Figs. 1 to 3, the distal ends of the pair of finger members 161 and 162 are close to each other. At this time, the pair of finger members 161 and 162 are maintained at a position close to the pushing portion 150 in the Z-axis direction. This state is called the "closed state" of the operation member 170.

When the operation member 170 is rotated toward the other direction from the state illustrated in Figs. 1 to 3, each of the pair of finger members 161 and 162 are rotated so as to force the distal ends thereof to separate from each other. At this time, the pair of finger members 161 and 162 slide away from the pushing portion 150 in the Z-axis direction. This state is called the "open state" of the operation member 170.

After the operation member 170 is turned to the "open state", the flange of the plunger rod is arranged between the pushing portion 150 and the gripping portion 160, and then the operation member 170 is returned to the "closed state", whereby the flange of the plunger rod is clamped and fixed in the thickness direction (the Z-axis direction). Thus, the pushing portion 150 and the gripping portion 160 function as a "clamping mechanism" that clamps and fixes the flange of the plunger rod in the thickness direction.

The pushing portion 150 and the gripping portion 160 are driven by the drive unit of the main body 110 to slide together with the slide shaft 140 in the axial direction (the Z-axis direction) of the slide shaft 140. The axial direction of the slide shaft 140 is identical to the axial direction of the plunger rod. Thus, the drive unit of the main body 110 and the slide shaft 140 function as a "moving mechanism" that moves the "clamping mechanism" along the axial direction of the plunger rod.

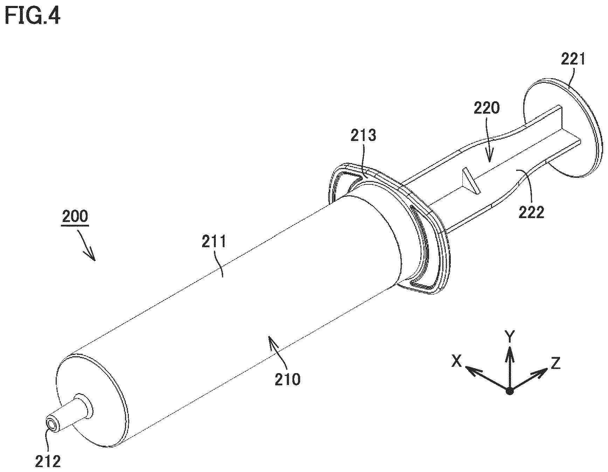

Fig. 4 is a perspective view illustrating an appearance of a syringe to be installed in the syringe pump 100. As illustrated in Fig. 4, the syringe 200 includes a barrel 210 and a plunger rod 220. The barrel 210 has a cylindrical housing member 211 that houses a liquid medicine therein. A liquid medicine inlet/outlet 212 is provided at a front end of the barrel 210. An opening is provided at a rear end of the barrel 210, and a flange 213 is formed around the opening.

The barrel 210 is made of a transparent or translucent resin such as polypropylene. Lubricating oil such as silicone oil may be applied to an inner peripheral surface of the barrel 210.

A gasket (not shown) is provided at the distal end of the plunger rod 220, and is configured to be in slide contact with the inner peripheral surface of the barrel 210. A flange 221 is provided at the rear end of the plunger rod 220. The plunger rod 220 includes a shaft 222 that connects the gasket and the flange 221.

Hereinafter, the configuration and operation of each of the pushing portion 150, the gripping portion 160 and the operation member 170 will be described in detail.

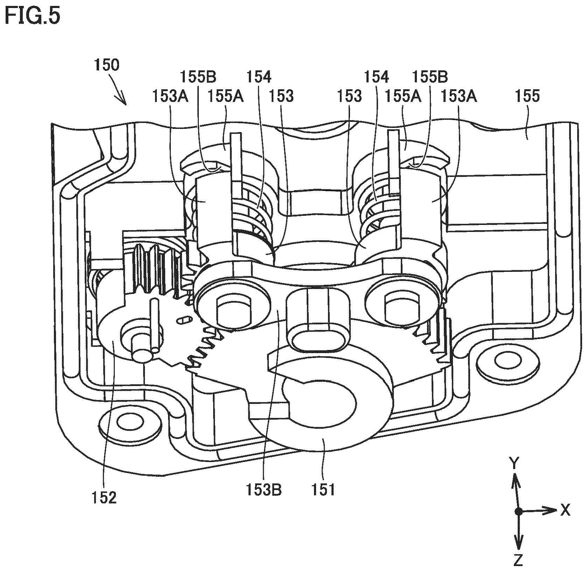

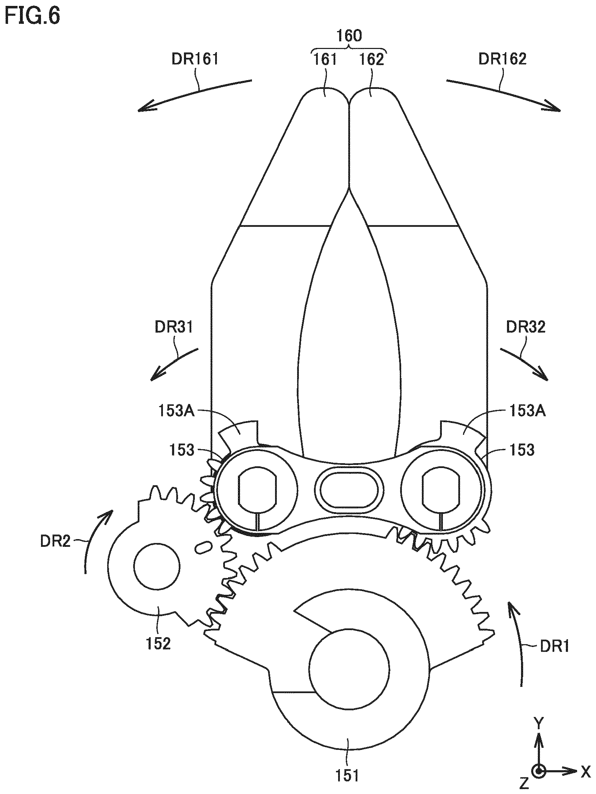

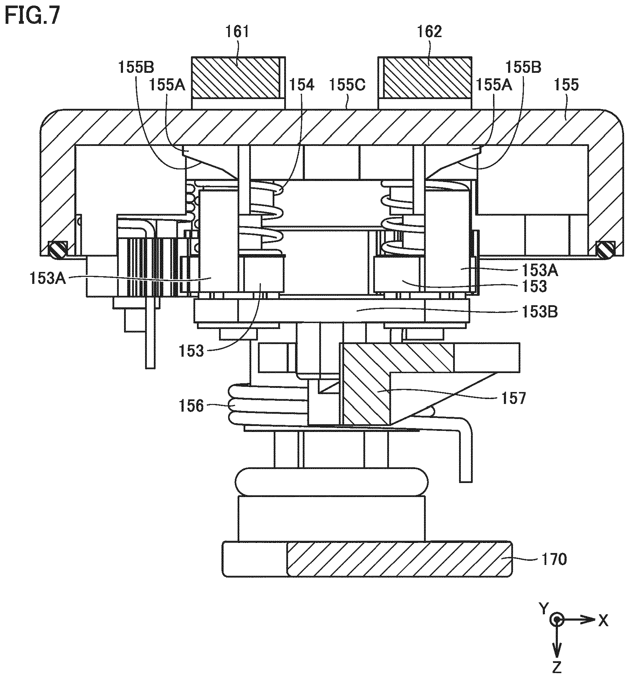

Fig. 5 is a perspective view illustrating an internal structure of the pushing portion 150, Fig. 6 is a view illustrating a transmission mechanism that transmits an operation force from the operation member 170 to the gripping portion 160, and Fig. 7 is a cross-sectional view of the pushing portion 150 cut along the Y-axis direction.

As illustrated in Figs. 5 to 7, the pushing portion 150 includes a first member 151, a second member 152, a third member 153, a bias member 154, a casing 155, a bias member 156, and a rotating member 157. The casing 155 houses the first member 151, the second member 152, the third member 153, the bias member 154, the bias member 156, and the rotating member 157.

In Figs. 5 to 7, in order to simplify the internal structure of the pushing portion 150, a portion of the casing 155 is not shown.

At the time of installing the syringe 200 in the syringe pump 100, the operation member 170 is firstly rotated. Thereby, the distal ends of the pair of finger members 161 and 162 are separated from each other.

More specifically, the rotational operation force generated by the operation member 170 is transmitted to the first member 151 through the rotating member 157. Thereby, the first member 151 rotates in the direction indicated by an arrow DR1 in Fig. 6. Due to the rotation of the first member 151, the second member 152 which is engaged with the first member 151 rotates in the direction indicated by an arrow DR2.

The teeth provided on a left part of the third member 153 mesh with the teeth of the second member 152, and the teeth provided on a right part of the third member 153 mesh with the teeth of the first member 151. Due to the rotation of the first member 151 and the second member 152, the left part rotates in the direction indicated by an arrow DR31, and the right part of the third member 153 rotates in the direction indicated by an arrow DR32. The left part of the third member 153 is coupled to the finger member 161, and the right part of the third member 153 is coupled to the finger member 162. Therefore, due to the rotation of the third member 153, the pair of finger members 161 and 162 rotate in the direction indicated by an arrow DR161 and in the direction indicated by an arrow DR162, respectively. Thereby, the distal ends of the pair of finger members 161 and 162 are separated from each other, achieving the "open state".

In the "open state", the flange 221 of the plunger rod 220 is arranged between the pair of finger members 161, 162 and the casing 155. Then, by rotating the operation member 170 in the reverse direction, the distal ends of the pair of finger members 161 and 162 become close to each other. In other words, by rotating the first member 151, the second member 152 and the third member 153 in a direction opposite to the direction of switching the operation member 170 to the "open state", the distal ends of the pair of finger members 161 and 162 become close to each other, achieving the "closed state".

The first member 151, the second member 152, and the third member 153 constitute a "transmission mechanism" that transmits an operation force from the operation member 170 to the gripping portion 160.

As illustrated in Figs. 5 to 7, the third member 153 includes a protrusion 153A (abutment member) that protrudes toward the inner surface of the casing 155, and a connection member 153B that connects the left part and the right part of the third member 153. The casing 155 is provided with a protrusion 155A that protrudes toward the third member 153. The protrusion 153A of the third member 153 comes into contact with the protrusion 155A of the casing 155, thereby preventing the third member 153 from moving in the Z-axis direction (the direction away from the operation member 170).

A tapered face 155B is formed at the tip of the protrusion 155A, and the tapered face 155B is configured to change a protruding amount of the protrusion 155A in the circumferential direction of the protrusion 155A. The casing 155 has a pressing face 155C facing the pair of finger members 161 and 162.

The bias member 154 biases the third member 153 and the gripping portion 160 toward the operation member 170 (in the +Z direction). The bias member 156 biases the first member 151 away from the operation member 170 (in the -Z direction). In other words, the first member 151, the second member 152, and the third member 153 are supported on the casing 155 with the bias members 154 and 156 interposed therebetween in the Z-axis direction.

In the "closed state" of the operation member 170, the protrusion 153A of the third member 153 is in contact with the protrusion 155A of the casing 155 at the largest protruding amount (the largest height). Therefore, in the "closed state", the third member 153 and the finger members 161 and 162 are prevented from moving in the -Z direction (toward the upper side of Fig. 7). As a result, the finger members 161 and 162 are maintained at a position close to the pressing face 155C.

The tapered face 155B is formed such a manner that the height of the protrusion 155A of the casing 155 in contact with the protrusion 153A of the third member 153 gradually decreases as the left part and the right part of the third member 153 rotate in the direction indicated by the arrow DR31 and the direction indicated by the arrow DR32, respectively. Thus, the third member 153 and the finger members 161 and 162 become movable in the -Z direction (toward the upper side of Fig. 7) as the state is switched from the "closed state" to the "open state". As a result, the finger members 161 and 162 are slid to a position separated from the pressing face 155C, which allows the flange 221 of the plunger rod 220 to be arranged between the finger members 161 and 162 and the pressing face 155C.

When the pair of finger members 161 and 162 are returned from the "open state" to the "closed state", the third member 153 and the finger members 161 and 162 are prevented from moving in the -Z direction (toward the upper side of Fig. 7) again, and the finger members 161 and 162 are returned to the position close to the pressing face 155C.

Through the operations described above, the flange 221 of the plunger rod 220 can be clamped and fixed in the thickness direction of the flange 221. In the "open state", the finger members 161 and 162 are slid to a position separated from the pressing face 155C. The slide amount changes in response to the opening angle of the finger members 161 and 162, in other words, the rotation angle of the operation member 170. Therefore, even when the thickness of the flange 221 varies depending on the size of the syringe 200, it is possible to fix the flange 221 by appropriately adjusting the gap between the pressing face 155C and the finger members 161 and 162. Further, it is possible to install the syringe 200 in a state where the gap between the finger members 161 and 162 and the pressing face 155C is relatively wide. Thus, according to the syringe pump 100 of the present embodiment, it is possible to improve the efficiency of fixing the flange 221.

After the flange 221 of the plunger rod 220 is clamped and fixed, the pair of finger members 161 and 162 are biased in the +Z direction by a biasing force from the bias member 154. However, when a negative pressure is generated in an enclosed path to which the syringe 200 is connected, a force in the -Z direction which is larger than the biasing force from the bias member 154 in the +Z direction may act on the plunger rod 220.

According to the syringe pump 100 of the present embodiment, when the flange 221 is fixed, the protrusion 153A of the third member 153 and the protrusion 155A of the casing 155 are brought into contact with each other, whereby the finger members 161 and 162 (the gripping portion 160) are prevented from moving in the -Z direction (toward the upper side of Fig. 7), which makes it possible to prevent the plunger rod 220 from moving unintendedly even under a negative pressure condition. The protrusion 153A of the third member 153 and the protrusion 155A of the casing 155 function as a "locking mechanism" that locks the movement of the gripping portion 160 in the Z-axis direction after the flange 221 is fixed.

Each configuration described in the present embodiment may be summarized in the following. The syringe pump 100 is applied to the syringe 200 that includes the barrel 210 and the plunger rod 220 which is fitted inside the barrel 210. The flange 221 is provided at a rear end of the plunger rod 220. The syringe pump 100 includes the pushing portion 150 and the holding portion 160 (constituting a clamping mechanism) which clamp and fix the flange 221 of the plunger rod 220 in the thickness direction of the flange 221. The pushing portion 150 includes the pressing face 155C facing a rear end face of the flange 221. The gripping portion 160 is movable in the thickness direction of the flange 221 (the Z-axis direction) and connected to the pushing portion 150 so as to form a gap with the pressing face 155C in the Z-axis direction, and is configured to work in cooperation with the pushing portion 150 so as to fix the flange 221. The pushing portion 150 locks the movement of the gripping portion 160 in a direction away from the pressing face 155C with the flange 221 being fixed.

The pushing portion 150 includes the first member 151, the second member 152 and the third member 153 (constituting a transmission mechanism) for transmitting an operation force from the operation member 170 to the gripping portion 160, and the casing 155 which forms the pressing face 155C and houses the first member 151, the second member 152 and the third member 153. The movement of the gripping portion 160 in the Z-axis direction is locked when the protrusion 153A (an abutment member) provided on the third member 153 abuts against the tapered face 155B formed inside the casing 155.

The gripping portion 160 includes a pair of finger members 161 and 162 rotatable about the Z axis with respect to the pushing portion 150. The third member 153 (a rotating member) rotates together with the finger members 161 and 162.

The bias member 154 biases the third member 153 in the +Z direction to draw the gripping portion 160 toward the pressing face 155C.

It should be understood that the embodiments disclosed herein have been presented for the purpose of illustration and description but not limited in all aspects. It is intended that the scope of the present invention is not limited to the description above but defined by the scope of the claims and encompasses all modifications equivalent in meaning and scope to the claims.

### REFERENCE SIGNS LIST

100: syringe pump; 110: main body; 120: syringe mounting member; 121: concave member; 122: syringe clamp; 130: barrel flange retainer; 140: slide shaft; 150: pushing portion; 151: first member; 152: second member; 153: third member; 153A: protrusion; 153B: connection member; 154: bias member; 155: casing; 155A: protrusion; 155B: tapered face; 155C: pressing face; 156: bias member; 157: rotating member; 160: gripping portion; 161, 162: finger member; 170: operation member; 200: syringe; 210: barrel; 211: housing member; 212: liquid medicine inlet/outlet; 213: flange; 220: plunger rod; 221: flange; 222: shaft

## Claims

1. A syringe pump to be applied to a syringe that includes a barrel and a plunger rod which is fitted inside the barrel, a flange being provided at a rear end of the plunger rod, the syringe pump comprising:
a clamping mechanism which clamps and fixes the flange of the plunger rod in a thickness direction of the flange,
the clamping mechanism including:
a pushing portion which includes a pressing face facing a rear end face of the flange; and
a gripping portion which is movable in the thickness direction and connected to the pushing portion so as to form a gap with the pressing face in the thickness direction, and is configured to work in cooperation with the pushing portion so as to fix the flange,
the pushing portion including a locking mechanism which locks the movement of the gripping portion in a direction away from the pressing face with the flange being fixed.

2. The syringe pump according to claim 1, further comprising an operation member for operating the gripping portion, wherein
the pushing portion further includes a transmission mechanism which transmits an operation force from the operation member to the gripping portion, and a casing which forms the pressing face and houses the transmission mechanism, and
the locking mechanism is formed between the transmission mechanism and the casing.

3. The syringe pump according to claim 2, wherein
the locking mechanism includes a tapered face formed on the casing, and an abutment member provided on the transmission mechanism and configured to abut against the tapered face.

4. The syringe pump according to claim 3, wherein
the gripping portion includes a finger member rotatable about an axis in the thickness direction with respect to the pushing portion,
the transmission mechanism includes a rotating member which rotates together with the finger member, and
the abutment member is formed on the rotating member.

5. The syringe pump according to any one of claims 2 to 4, wherein
the pushing portion further includes a bias member configured to bias the transmission mechanism so as to draw the gripping portion toward the pressing face.
